# EUROPEAN PATENT APPLICATION

(11) **EP 2 807 975 A1**
(43) Date of publication of application: **03.12.2014**
(21) Application number: 13741403.3
(22) Date of filing: 23.01.2013
(51) Int. Cl.: A61B 5/00, A61B 5/0245, A61B 5/08

(54) **METHOD FOR MONITORING ANIMAL RESPIRATION AND/OR PULSE CHANGES**

(30) Priority: 23.01.2012 JP 2012011026; 10.05.2012 JP 2012108829
(71) Applicant: UEDA JAPAN RADIO CO., LTD., Ueda-shi, Nagano 386-8608 (JP); Toyota Jidosha Kabushiki Kaisha, Toyota-shi, Aichi 471-8571 (JP)
(72) Inventor: OHYA, Shigemasa, Ueda-shi Nagano 386-8608 (JP); SHIGETO, Kazuhide, Aichi 4718571 (JP); KIKUCHI, Hirokasu, Aichi 4718571 (JP)
(74) Representative: Banzer, Hans-Jörg
(86) International application number: PCT/JP2013/051336
(87) International publication number: WO 2013/111785

(57) **Abstract**

A method of monitoring variation in either or both respiration and heartbeat of an animal comprising the steps of vibrating a piezoelectric vibrator by continuously or intermittently applying an AC voltage of a frequency corresponding to a natural resonance frequency of the vibrator to the piezoelectric vibrator under such condition that the vibrator is placed directly or indirectly in contact with the surface of the animal's body; collecting a current generated by the vibrator under vibration; and calculating an impedance of the vibrator from a value of the current and a value of the AC voltage applied to the vibrator so as to continuously or intermittently detect variation of the impedance by lapse of time is effective to monitor variation of respiration and/or heartbeat of animals including humans who are in sleep or under exercise and take a variety of positions with high sensitivity.

## Description

### FIELD OF THE INVENTION

The present invention relates to a method for monitoring variation in respiration and/or heartbeat (pulse) of an animal including human being, and further relates to a device for monitoring variation in respiration and/or heartbeat which is favorably employable for performing said method.

### BACKGROUND OF THE INVENTION

In medical facilities, it is required to continuously check and manage physical conditions of patients. Particularly, it is mostly required to continuously check variation (change) in respiration and/or heartbeat (pulse) of the patients so as to timely take appropriate care of the patients when any abnormal conditions occur with respect to the respiration or heartbeat of the patient. As for exercising people, it is desired to immediately inform them when abnormal conditions occur in their respiration or heartbeat so that they can immediately discontinue the exercise and can be kept from being injured.

As for devices for monitoring respiration and/or heartbeat of human being, the below-described devices are known.

JP 2003-339652 A describes a device for measuring heartbeat/respiration which comprises a sensor having an electrode on both surface sides which is set under such conditions that the sensor is placed under pressure of human being and a measuring circuit capable of measuring heartbeat and/or respiration from output of the sensor, in which the sensor is composed of deformable elastic dielectric material sandwitched by a couple of electroconductive members, and the measuring circuit comprises a resonance circuit which is capable of resonating with the sensor as an oscillating condenser and a processing circuit capable of detecting variation of the oscillating frequency of the resonance circuit and calculating the number of heartbeat and/or respiration from the variation of the frequencies of the heartbeat and/or respiration. According to this patent publication, when a man lies down on the horizontally placed sensor of the device, the dielectric material of the sensor elastically deforms in the thickness direction in response to the periodical variation of the pressure applied to the sensor which is caused by heartbeat and respiration of the lying body. As a result, the distance between the electrodes varies and the electrostatic capacity of the sensor varies. The variation of the electrostatic capacity of the sensor can be detected and converted into variation of the oscillating frequency of the resonance circuit to give the number of heartbeat and respiration.

JP 2006-129933 describes a device comprising a deformable oscillation transmitting plate having plural through-holes and a sensor for heartbeat-respiration which has a piezoelectric transducer attached to the oscillation transmitting plate. This patent publication describes that the heartbeat and respiration of a newborn baby by placing the device between a bed and a sheet on which the newborn baby lies, because the oscillating-transmitting plate oscillates in response to the heartbeat and respiration of the baby and the oscillation is transmitted to the piezoelectric transducer in which the oscillation is converted into electric signals corresponding to the deformation of the transducer caused by the oscillation.

As is described from the descriptions of the above-mentioned patent publication, previously known devices for monitoring human heartbeat or respiration should be placed under a lying human being, and hence the device has to employ a large size sensor or a great number of sensors.

On the other hand, if it is needed to monitor the respiration or heartbeat of a standing person (including person taking sitting positions other than the lying position), it is desired that the sensor is so small as not to disturb movement of the standing person. Also the small-sized sensor is desired for monitoring respiration and/or heartbeat of an animal other than human beings, such as a dog or a cat, so as not to disturb the movement of the animal.

However, since the sensitivity of the known monitoring systems which function by detecting variation of the thickness of the piezoelectric transducer (i.e., piezoelectric vibrator) is not high, it is not able to employ a sufficiently small sized sensor.

### SUMMARY OF THE INVENTION

Accordingly the object of the invention is to provide a new method for monitoring variation of respiration or heartbeat of animals including human being, who are lying or exercising and taking various positions, with high sensitivity. Particularly, the object of the invention is to provide a system employing a small sized sensor. A further object of the invention is to provide a device employable for performing the new monitoring system.

The inventors of the present invention have found that the impedance, namely, variation of a resonance sharpness (Q value), of a vibrator that is placed directly or indirectly in contact with the surface of the body of the animal such as human being under pressure and vibrates under continuous or intermittent application of an AC voltage of a frequency corresponding to the natural resonance frequency of the vibrator varies with high sensitivity under oscillation caused by respiration and heartbeat of the animal. The impedance of the vibrator is calculated from a value of the current collected from the vibrating vibrator and a value of the AC voltage applied to the vibrator.

The inventors have further found that the variation of respiration and/or heartbeat of the animal can be detected with high sensitivity by detecting the variation of the impedance of the piezoelectric vibrator with passage of time.

The present invention has been made based on the above-mentioned findings.

Accordingly, the invention resides in a method for monitoring variation in respiration and/or heartbeat of an animal which is performed by vibrating a piezoelectric vibrator placed directly or indirectly in contact with the surface of the body of the animal under pressure by continuously or intermittently applying to the vibrator an AC voltage of a frequency corresponding to the natural resonance frequency of the vibrator and detecting variation of impedance of the vibrator caused by the respiration and/or heartbeat of the animal with the passage of time.

The invention can be described as a method for monitoring variation in either or both respiration and heartbeat of an animal which comprises the steps of:
vibrating a piezoelectric vibrator placed directly or indirectly in contact with the surface of the body of the animal under pressure by continuously or intermittently applying to the vibrator an AC voltage of a frequency corresponding to the natural resonance frequency of the vibrator;
detecting a current generated by the vibrator under vibration; and
calculating impedance of the vibrator from a value of the current and a value of the AC voltage applied to the vibrator to detect continuously or intermittently variation of impedance with the passage of time.

The preferred embodiments of the invention are described below.
(1) The animal is a human being, a dog, a cat, a horse, a cow, a monkey, one of other domestic animals, or one of other animals kept at zoological gardens.
(2) The monitoring is made on variation of a frequency of respiration of the animal, and the variation of frequency is monitored by detecting plural peaks corresponding to minimum values of impedance which are periodically observed on a graph showing the variation of impedance, and a space in time between the adjoining peaks is assigned to the frequency of respiration.
(3) In the method of (2) above, variation of a difference of height or depth between the adjoining peaks is further monitored.
(4) The monitoring is made on variation of a strength of respiration of the animal, and the variation of strength is monitored by detecting plural peaks corresponding to minimum values of impedance which are periodically observed on a graph showing the variation of impedance, and a difference in height or depth between the adjoining peaks is assigned to the variation of strength of the respiration.
(5) The monitoring is made on variation of a frequency of heartbeat of the animal, and the variation of the frequency is monitored by detecting second plural peaks which are observed in the area between the plural first peaks corresponding to the minimum values of impedance and of which amplitudes are at a second level, these peaks being periodically observed in a graph showing the variation of impedance, and a space in time between the adjoining second peaks is assigned to the frequency of heartbeat.
(6) In the method of (5) above, variation of a difference of height or depth between the adjoining second peaks is further monitored.
(7) The monitoring is made on variation of a strength of heartbeat of the animal, and the variation of the strength is monitored by detecting second plural peaks which are observed in the area between the plural first peaks corresponding to the minimum values of impedance and of which amplitudes are at a second level, these peaks being periodically observed in a graph showing the variation of impedance, and a difference in height or depth between the adjoining second peaks is assigned to the variation of strength of heart beat.
(8) The piezoelectric vibrator is placed on the surface of the body of a standing animal.
(9) The piezoelectric vibrator is placed on the surface of the body of an exercising animal.
(10) The piezoelectric vibrator is placed at a site receiving the weight of the animal.

The invention further resides in a device for monitoring variation in either or both respiration and heartbeat of an animal which comprises a piezoelectric vibrator, an impedance-detecting circuit electrically connected to the piezoelectric vibrator, that is capable of applying continuously or intermittently to the vibrator an AC voltage of a frequency corresponding to the natural resonance frequency of the vibrator, detecting a current generated by the vibrator under vibration and calculating impedance of the vibrator from a value of the current and a value of the AC voltage applied to the vibrator, and a computing circuit for detecting continuously or intermittently variation of impedance with the passage of time. The device can be equipped with a display for graphically displaying the variation of impedance.

### EFFECTS OF THE INVENTION

The method and device of the invention can make possible to monitor with high sensitivity variation of the respiration and/or heartbeat of an animal lying or standing, particularly an exercising animal.

The variation of impedance of vibrating vibrator to which vibration of respiration or heartbeat of an animal is applied is prominently larger than the variation of electric vibration occurring in a piezoelectric vibrator to which vibration of respiration or heartbeat of an animal is applied.

Accordingly, the method and device of the invention show increased sensitivity, and hence a small sized piezoelectric vibrator (i.e., sensor) can be utilized.

### BRIEF DESCRIPTION OF DRAWINGS

Fig. 1 is a block diagram of one example of the device for monitoring variation of respiration and/or heartbeat of an animal designed for monitoring variation of respiration and/or heartbeat of a sleeping person, which embodies the invention.
Fig. 2 an enlarged section of an example of sheet in which plural piezoelectric vibrators are arranged and which is employable in the device of Fig. 1.
Fig. 3 an enlarged section of another example of sheet in which plural piezoelectric vibrators are arranged and which is employable in the device of Fig. 1.
Fig. 4 is a block diagram of one example of the device for monitoring variation of respiration and/or heartbeat of an animal designed for monitoring variation of respiration and/or heartbeat of an exercising person, which embodies the invention.
Fig. 5 is a graph showing variation of human respiration and heartbeat detected by the use of a monitoring device of Fig. 4.

### EMBODIMENTS OF THE INVENTION

The method of the invention for monitoring variation of respiration and/or heartbeat can be mainly applicable to human beings. However, the method can be applicable to animals other than human beings. Examples of the animals other than human beings include domestic animals and animals kept at zoological gardens or farms, such as dogs, cats, horses, cows and monkeys.

The method of invention for monitoring variation of respiration and/or heartbeat of animals can be performed by vibrating a piezoelectric vibrator (or piezoelectric oscillator) placed directly or indirectly in contact with the surface of the body of the animal under pressure by continuously applying to the vibrator a predetermined AC voltage, and detecting variation of an impedance caused by respiration and/or heartbeat of the animals. The piezoelectric vibrator can be placed directly or indirectly, for instance, via clothes, on the surface of the body of the animal. A sheet can be placed between the piezoelectric vibrator and the surface of the body of the animal. The piezoelectric vibrator can be placed in a case and then attached to the surface of the animal body. The sheet and case can be made of material with any form and size under such condition that the sheet and case do not disturb transmittance of the vibration occurring in the animal by respiration and/or heartbeat to the piezoelectric vibrator. It is preferred that the sheet and case do not transmit the ultrasonic vibration generated by the piezoelectric vibrator to the whole area of the animal. Examples of the material for the sheet and case include resin (e.g., epoxy resin), rubber (e.g., hard rubber), cork, and cured material. These materials can contain substances that absorb or reflect ultrasonic wave. The term of "under pressure" is used to mean that the piezoelectric vibrator is placed in contact with the surface of the body to the extent that the current generated by the piezoelectric vibrator shows periodical variation of impedance upon receipt of variation of pressure caused by the animal's respiration and heartbeat.

When the variation of the respiration and/or heartbeat of a sleeping animal is monitored, the piezoelectric vibrator is preferably placed in such position to receive the weight of the animal's body. In addition, it is preferred to arrange plural piezoelectric vibrators so as to keep the moving body in contact with any one of the piezoelectric vibrators. If the variation of the respiration and/or heartbeat of a sleeping animal or an exercising animal, the piezoelectric vibrator is preferably fixed to the animal's body by means of fixing aids such as belts or adhesive tapes. Examples of the piezoelectric vibrators include electro-striction vibrators made of ferroelectric ceramic materials such as quartz crystal, lead titanate zirconate (PZT) and barium titanate. There are no specific limitations with respect to the shape of the piezoelectric vibrator. Therefore, the piezoelectric vibrator may be in the form of rectangular plate or disc. The size of the piezoelectric vibrator can be generally in the range of 1 to 50 mm, preferably in the range of 5 to 10 mm, in terms of diameter.

The AC voltage applied to the piezoelectric vibrator is an AC voltage of a frequency corresponding to the natural resonance frequency of the piezoelectric vibrator. The frequency corresponding to the natural resonance frequency of the piezoelectric vibrator means a frequency in the range of ± 10% (preferably± 5%) of the natural resonance frequency. The frequency of the AC voltage to be applied to the piezoelectric vibrator is generally in the range of 20 kHz to 10 MHz, preferably in the range of 20 kHz to 5 MHz, more preferably in the range of 20 kHz to 1 MHz, further preferably in the range of 20 to 500 kHz, most preferably in the range of 20 to 100 kHz. The voltage of the AC voltage to be applied to the piezoelectric vibrator is generally not higher than 5 V in terms of effective voltage, preferably in the range of 1 to 3 V.

When a piezoelectric vibrator is placed on a surface of an animal body and is caused to vibrate, the impedance of the piezoelectric vibrator varies depending upon the vibration caused by respiration and/or heartbeat of the animal. In the present invention, the variation of respiration and/or heartbeat of the animal is monitored by detecting the variation of the impedance from the vibrator with the passage of time. For instance, the variation of frequency of respiration and/or heartbeat of the animal can be monitored by detecting the variation of frequency of the impedance from the vibrator. The variation of frequency of the impedance means a time interval between the times when the increasing impedance decreases or times when the decreasing impedance increases. Further, the variation of strength of respiration and/or heartbeat of the animal can be monitored by detecting the values of variation of the impedance. The values of variation of the impedance means a difference of impedance between the values shown at the times when the increasing impedance decreases or at the times when the decreasing impedance increases.

The variation curve of the impedance from the piezoelectric vibrator with passage of time composes the variation of the impedance caused by respiration and the variation of the impedance caused by heartbeat. Hence, it is preferred to monitor the variation caused by respiration and the variation caused by heartbeat independently, so as to monitor the frequency and strength of respiration and heartbeat independently. Since the range of variation of the impedance caused by respiration apparently differs from the range of variation of the impedance caused by heartbeat, the variations of the impedance with the passage of time can be easily separated to detect the variation caused by respiration and the variation caused by heartbeat independently.

The impedance (Z) of a piezoelectric vibrator can be calculated (or measured) from the voltage (V₁) of the AC voltage applied to the vibrator and the current (I) generated in the vibrator utilizing the equation of Z=V₁/I.

The device of the invention for monitoring variation in respiration and/or heartbeat of an animal comprises a piezoelectric vibrator, an impedance-detecting circuit electrically connected to the piezoelectric vibrator, and a computing circuit.

The impedance-detecting circuit is capable of applying continuously or intermittently to the vibrator an AC voltage of a frequency corresponding to the natural resonance frequency of the vibrator, detecting and measuring a current generated by the vibrator under vibration, and calculating impedance of the vibrator from a value of the current and a value of the AC voltage applied to the vibrator. The computing circuit is capable of detecting continuously or intermittently variation of impedance with the passage of time.

The piezoelectric vibrator, impedance-detecting circuit and computing circuit can be connected to each other with wires or without wires.

The impedance detected by the impedance-detecting circuit as well as the variation of the impedance with the passage of time detected by the computing circuit can be transmitted to a monitor room in which a staff such as a medical doctor or a nursing person are in attendance for monitoring the variation of respiration and/or heartbeat of the patient.

Otherwise, a sensor comprising a piezoelectric vibrator and a wireless transmitter capable of transmitting the impedance of the vibrator can be attached to a swimmer or climber for transmitting the body conditions of the swimmer or climber.

It is preferred that the monitoring device of the invention is equipped with a monitor for displaying the variation of impedance as the variation of respiration and/or heartbeat. Further, the device may have a system comprising a memory in which a normal range for the variation of impedance is recorded and a computer capable of checking if the detected variation of impedance is outside the normal range and transmitting a warning signal to the animal (patient) or a monitoring person. The normal range for the variation of impedance recorded in the computer can be rewritten to record a freshly checked normal range for the variation observed in an athlete starting his exercise. The monitoring device may be equipped with a system for separating the detected variation of impedance with passage of time into the variation of respiration and the variation of heartbeat.

The monitoring device of the invention is further described with reference to the attached drawings.

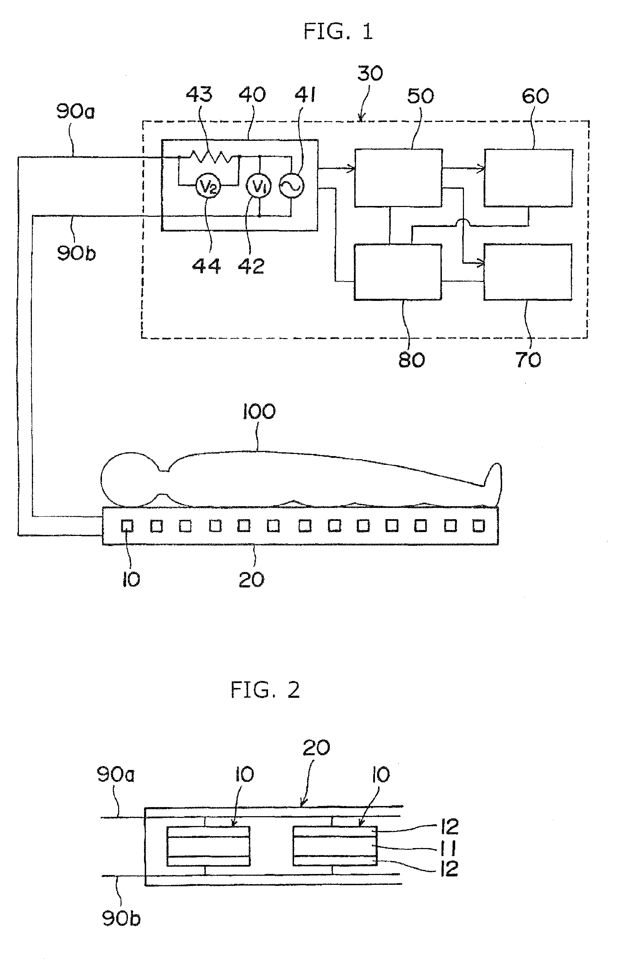

Fig. 1 is a block diagram of one example of the device of the invention for monitoring variation of respiration and/or heartbeat of an animal designed for monitoring variation of respiration and/or heartbeat of a sleeping person. Each of Fig. 2 and Fig. 2 is an enlarged section of an example of sheet 20 in which plural piezoelectric vibrators 10 are arranged and which is employable in the device of Fig. 1.

In Fig. 1, the monitoring device comprises a sheet 20 placed under the human body 100 and a data processor 30. The sheet 20 comprises plural piezoelectric vibrators 10 arranged therein.

The data processor 30 is electrically connected to the plural piezoelectric vibrators 10 via lead wires 90a, 90b, and comprises an impedance-measuring circuit 40 electrically connected to the lead wires 90a, 90b, a computing circuit 50 connected to the impedance-measuring circuit 40 for detecting variation measured in the impedance-measuring circuit 40, a display 60 electrically connected to the computing circuit 50 for displaying the variation of impedance with the passage of time detected in the computing circuit 50 as the variation of respiration and/or heartbeat, an alarm 70 electrically connected to the computing circuit 50 for emitting warning signals in accordance with the instructions transmitted from the computing circuit 50, and a power source 80 for supplying electric power to each of the impedance-measuring circuit 40, computing circuit 50, display 60 and alarm 70.

The impedance-measuring circuit 40 comprises an oscillator 41 for generating an AC voltage having a predetermined frequency, a voltmeter 42 for detecting a voltage (i.e., voltage applied to the piezoelectric vibrator 10) generated between the lead wire 90a and lead wire 90b, a resistor 43 placed in the lead wire 90a, a voltmeter 44 for detecting the voltage generated between the both ends of the resistor 43.

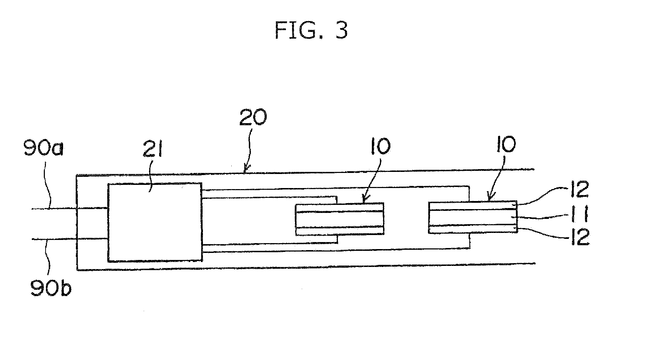

As is seen from Fig. 2 and Fig. 3, the piezoelectric vibrator 10 is composed of a piezoelectric body 11 and electrodes 12 placed to both ends of the piezoelectric body 11. The piezoelectric vibrator 10 is arranged in the sheet 20 under such conditions that the surface of the electrode 12 are aligned in parallel with the surface of the sheet 20. The plural piezoelectric vibrators 10 arranged in the sheet 20 can be connected to the lead lines 90a, 90b in parallel with each other. Otherwise, a switching means 21 can be placed for switching the connections between the plural piezoelectric vibrators 10 and the lead wires 90a, 90b.

The monitoring device in Fig. 1 can be employed for monitoring respiration and/or heartbeat of the human body 100 in the below-described manner.

An AC voltage of a predetermined frequency is generated in the oscillator 41 of the impedance-measuring circuit40, and the generated AC voltage is applied to the piezoelectric vibrator 10 via the lead wires 90a, 90b. Simultaneously with the application of the AC voltage, the voltmeter 42 functions to detect the voltage (V1) of the AS voltage applied to the piezoelectric vibrator 10. The voltmeter 44 detect the voltage (V2) between both ends of the resistor 43 (resistance: R), and calculate the current value (I) generated in the piezoelectric vibrator 10 according to the equation of I = V2/R. Then, the impedance of the piezoelectric vibrator (Z) is calculated from the voltage (V₁) of the AC voltage applied to the piezoelectric vibrator 10 and the current value (I) generated in the piezoelectric vibrator 10, according to the equation of Z = V₁/I.

The impedance-measuring circuit 40 transmits the detected impedance to the computing circuit 50. The computing circuit 50 detects from the received impedance the variation of the impedance with the passage of time which is caused by respiration and/or heartbeat of the human body 100, and transmits it to the display 60. The display 60 displays the received variation of impedance with the passage of time in the form of the variation of respiration and/or heartbeat of the human body 100. The computing-circuit 50 has the information for the normal range of the variation of impedance with the passage of time, and compares the detected variation of impedance with the normal range therein. If the detected variation of impedance is outside the normal range, the computing-circuit 50 sends a signal to the alarm 70. Then, the alarm 70 emits alarming signals such as a sound, a light or a vibration in accordance with the received signal, so as to transmit to the human 100 or its surrounding people to the effect that the human body is under abnormal conditions in connection with its respiration and/or heartbeat.

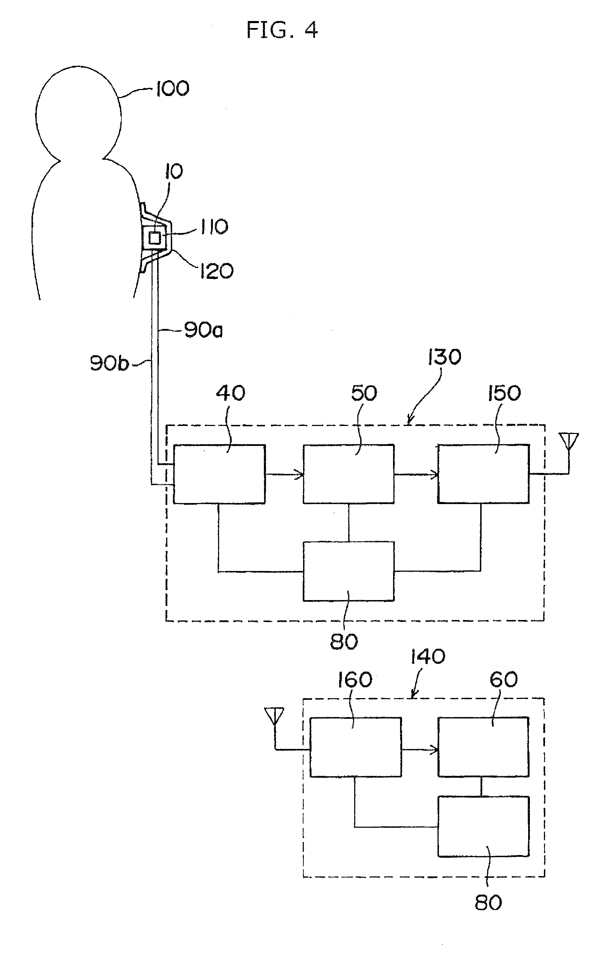

Fig. 4 is a block diagram of one example of the device for monitoring variation of respiration and/or heartbeat of an animal designed for monitoring variation of respiration and/or heartbeat of an exercising person. In Fig. 4, the monitoring device is illustrated in the same manner as in Fig. 1. Accordingly, the same numerals are assigned to the same circuits and means, and the detailed descriptions on the same circuits and means are omitted.

In Fig. 4, the monitoring device comprises a case 110 fixed to the human body 100 (which contains a piezoelectric vibrator 10), a data-transmitting device 130 connected to the piezoelectric vibrator 10 via lead wires 90a, 90b, and a data-receiving device 140 which is capable of receiving the data sent from the data-transmitting device 130.

The case 110 having the piezoelectric vibrator 10 is fixed to the human body 100 by means of fixing aids 120 such as belts or adhesive tapes. The data-transmitting device 130 comprises an impedance-measuring circuit 40 electrically connected to the lead wires 90a, 90b, a computing circuit 50 connected to the impedance-measuring circuit 40 and detecting the variation of impedance measured in the impedance-measuring circuit 40, an oscillator 150 connected to the computing circuit 50 and transmitting to a data-receiving device 140 the variation of impedance detected in the computing circuit 50, and a power source 80 for supplying electric power to each of the impedance-measuring circuit 40, computing circuit 50, and transmitter 150. The data-transmitting device 130 is ordinarily fixed to the human body or placed in a pocket of the clothes. The piezoelectric vibrator 10 can be combined with the data-transmitting device 130 and placed in one case. The data-receiving device 140 comprises a receiver 160 for receiving the variation of impedance from the transmitter 150, a display 60 connected to the receiver 160 and displaying the variation of impedance received by the receiver 160 as the variation of respiration and/or heartbeat, and a power source 80 supplying an electric power to the receiver 160 and display 60. The data-receiving device 140 is ordinarily set in a monitoring room in which a monitoring person resides. The data-transmitting device 130 and data-receiving device 140 can be arranged to transmit data from each other so that the data-transmitting device 130 can transmit the variation of impedance to the data-receiving device 140 when the data-transmitting device 130 receives a signal from the data-receiving device 140.

The monitoring of respiration and/or heartbeat of an exercising person can be performed using the device illustrated in Fig. 4.

The impedance-measuring circuit 40 of the data-transmitting device 130 detects impedance of the piezoelectric vibrator 10 and transmits it to the computing circuit 50. The computing circuit 50 detects the variation of impedance with the passage of time caused by the respiration and/or heartbeat of the human body 100 from the received impedance, and transmit the variation to the transmitter 150. The transmitter 150 transmits the received variation of impedance to the data-receiving device 140 placed in a monitoring room.

The receiver 160 of the data-receiving device 140 receives the variation of impedance with the passage of time from the transmitter 150 and transmits it to the display 60. The display 60 displays the received variation of impedance as variation of respiration and/or heartbeat of the human body 100. The monitoring person watches the variation of respiration and/or heartbeat presented on the display and gives such suggestion as discontinuation of exercise if he notes abnormal phenomenon.

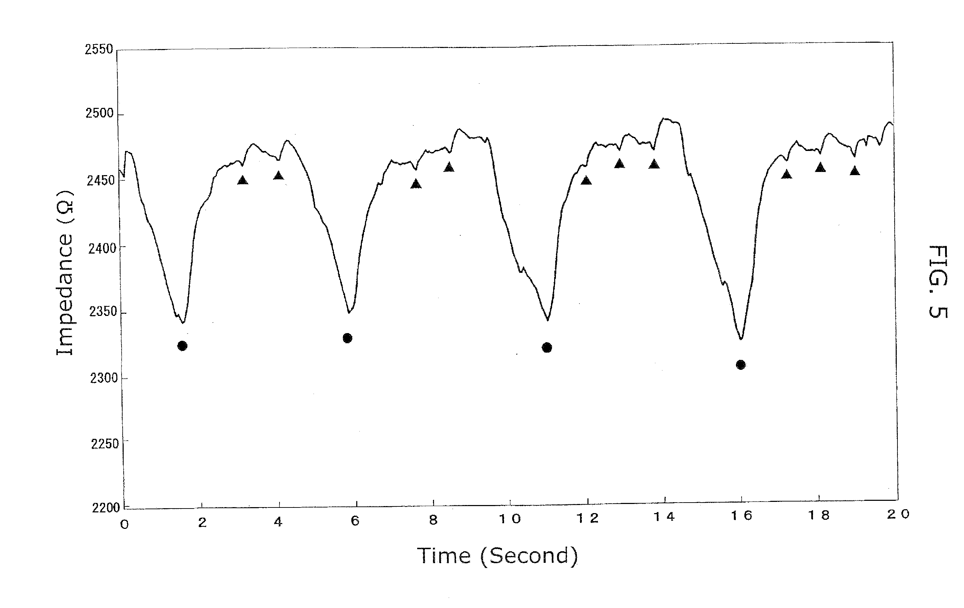

Fig. 5 is a graph showing variation of human respiration and heartbeat detected by the use of a monitoring device of Fig. 4.

In the graph of Fig. 5, the axis of abscissas indicates the time (unit: sec.), and the axis of ordinates indicates the impedance of the piezoelectric vibrator (unit: Ω). The frequency of the peaks (indicated by black round dots) is approx. 4.5 seconds which corresponds to the frequency of respiration of a human being. The frequency of the smaller peaks (indicated by black triangle dots) which are observed between the peaks indicated by black round dots is approx. One second which corresponds to the frequency of heartbeat of a human being.

### [Explanation of Symbols]

- 10: piezoelectric vibrator
- 11: piezoelectric body
- 12: electrode
- 20: sheet
- 21: switching means
- 30: data-processor
- 40: impedance-measuring circuit
- 41: oscillator
- 42: voltmeter
- 43: resistor
- 44: voltmeter
- 50: computing circuit
- 60: display
- 70: alarm
- 80: power source
- 90a, 90b: lead wire
- 100: human, human body
- 110: case
- 120: fixing aid
- 130: data-transmitting device
- 140: data-receiving device
- 150: transmitter
- 160: receiver

## Claims

1. A method for monitoring variation in either or both respiration and heartbeat of an animal which comprises the steps of:
vibrating a piezoelectric vibrator placed directly or indirectly in contact with the surface of the body of the animal under pressure by continuously or intermittently applying to the vibrator an AC voltage of a frequency corresponding to the natural resonance frequency of the vibrator;
detecting a current generated by the vibrator under vibration; and
calculating impedance of the vibrator from a value of the current and a value of the AC voltage applied to the vibrator to detect continuously or intermittently variation of impedance with the passage of time.

2. The method of claim 1, in which the animal is a human being.

3. The method of claim 1, in which the animal is a dog, a cat, a horse, a cow, a monkey, one of other domestic animals, or one of other animals kept at zoological gardens.

4. The method of claim 1, in which the monitoring is made on variation of a frequency of respiration of the animal, and the variation of frequency is monitored by detecting plural peaks corresponding to minimum values of impedance which are periodically observed on a graph showing the variation of impedance, and an interval of time between the adjoining peaks is assigned to the frequency of respiration.

5. The method of claim 4, in which variation of a difference of height or depth between the adjoining peaks is further monitored.

6. The method of claim 1, in which the monitoring is made on variation of a strength of respiration of the animal, and the variation of strength is monitored by detecting plural peaks corresponding to minimum values of impedance which are periodically observed on a graph showing the variation of impedance, and a difference in height or depth between the adjoining peaks is assigned to the variation of strength of the respiration.

7. The method of claim 1, in which the monitoring is made on variation of a frequency of heartbeat of the animal, and the variation of the frequency is monitored by detecting second plural peaks which are observed in the area between the plural first peaks corresponding to the minimum values of impedance and of which amplitudes are at a second level, these peaks being periodically observed in a graph showing the variation of impedance, and an interval of time between the adjoining second peaks is assigned to the frequency of heartbeat.

8. The method of claim 7, in which variation of a difference of height or depth between the adjoining second peaks is further monitored.

9. The method of claim 1, in which the monitoring is made on variation of a strength of heartbeat of the animal, and the variation of the strength is monitored by detecting second plural peaks which are observed in the area between the plural first peaks corresponding to the minimum values of impedance and of which amplitudes are at a second level, these peaks being periodically observed in a graph showing the variation of impedance, and a difference in height or depth between the adjoining second peaks is assigned to the variation of strength of heart beat.

10. The method of claim 1, in which the piezoelectric vibrator is placed on the surface of the body of a standing animal.

11. The method of claim 1, in which the piezoelectric vibrator is placed on the surface of the body of an exercising animal.

12. The method of claim 1, in which the piezoelectric vibrator is placed at a site receiving the weight of the animal.

13. A device for monitoring variation in either or both respiration and heartbeat of an animal which comprises a piezoelectric vibrator, an impedance-detecting circuit electrically connected to the piezoelectric vibrator, that is capable of applying continuously or intermittently to the vibrator an AC voltage of a frequency corresponding to the natural resonance frequency of the vibrator, detecting a current generated by the vibrator under vibration and calculating impedance of the vibrator from a value of the current and a value of the AC voltage applied to the vibrator, and a computing circuit for detecting continuously or intermittently variation of impedance with the passage of time.

14. The device of claim 13, which is equipped with a display for graphically displaying the variation of impedance.
